# EUROPÄISCHE PATENTSCHRIFT

(11) **EP 0 649 426 B1**
(45) Veröffentlichungstag und Bekanntmachung des Hinweises auf die Patenterteilung: **26.09.2001**
(21) Anmeldenummer: 93914694.0
(22) Anmeldetag: 22.06.1993
(51) Int. Cl.: C07F 9/32, C07K 5/06, C07F 9/48

(54) **INHIBITOREN RETROVIRALER PROTEASEN**
INHIBITORS OF RETROVIRAL PROTEASES
INHIBITEURS DE PROTEINASES RETROVIRALES

(30) Priorität: 24.06.1992 DE 4220566
(43) Veröffentlichungstag der Anmeldung: 26.04.1995
(73) Patentinhaber: Aventis Pharma Deutschland GmbH, 65929 Frankfurt am Main (DE)
(72) Erfinder: STOWASSER, Bernd, D-6114 Gro -Umstadt (DE); LI, Jian-Qi, 200083 Shanghai (CN); PEYMAN, Anuschirwan, D-6233 Kelkheim (DE); BUDT, Karl-Heinz, D-6233 Kelkheim (DE)
(86) Internationale Anmeldenummer: EP9301594
(87) Internationale Veröffentlichungsnummer: WO9400461

(56) Entgegenhaltungen:
- EP-A- 0 428 849
- EP-A- 0 435 059
- NATURE, Band 329, Nr. 6140, 15.-21. Oktober 1987, I. KATOH et al. "Inhibition of retroviral protease activity by an aspartyl pro- teinase inhibitor",
- BLUNDELL T. AND PEARL L.: "A second front against AIDS", NATURE, LONDON, 1989, Band 337, Nr. , Seiten 596 - 597

## Beschreibung

Die vorliegende Erfindung betrifft Substanzen, die die Wirkung retroviraler Proteasen hemmen, Verfahren zu ihrer Herstellung, ihre Verwendung sowie diese enthaltende Arzneimittel.

Die etiologische Ursache des "erworbenen Immunschwäche-Syndroms" (engl.: acquired immune deficiency syndrome (AIDS)) ist der sogenannte human immunodeficiency virus (HIV) (F. Barre-Sinoussi et al., Science 220, (1983), 868-870; R. C. Gallo et al., Science 224, (1984), 500 - 502; R.C. Gallo und L. Montagnier, Scient. Am. 259(4), (1988), 40 - 48. HIV ist ein Retrovirus und gehört in die Gruppe der Lentiviren (M. A. Gonda, F. Wong-Staal und R. C. Gallo, Science, 227, (1985), 173; P. Sonigo et al., Gell, 42, (1985), 369).

Die Aids Epidemie hat sich mittlerweile über nahezu alle Staaten mehr oder weniger ausgebreitet. Die Welt Gesundheits Organisation (WHO) schätzt die Zahl der infizierten Erwachsenen weltweit auf etwa 8 - 10 Millionen (Weekly Epidemiological Record, World Health Organization, Geneva, 1991, 66, 353 - 357). Von diesem haben bereits über 1 Millionen AIDS und eine weitere Million schwere infektionsbedingte Krankheiten entwickelt. Es wurden 1 Million über ihre Mütter infizierte Kinder geboren, von denen etwa die Hälfte bereits AIDS entwickelt haben oder gestorben sind. WHO rechnet für das Jahr 2000 mit etwa 30 - 40 Millionen Infizierter.

Die einzige bisher für die Indikation AIDS zugelassene Substanz Zidovudine (AZT) vermag das Leben der Patienten in vielen Fällen zu verlängern, besitzt jedoch ernste, toxische Nebeneffekte, die in vielen Fällen den Absatz der Therapie verlangen. Es wurden bereits Stämme von HIV entdeckt, die eine deutlich geringere Empfindlichkeit gegen AZT zeigten und somit zu einer Resistenzentwicklung führten. Weitere Ansatzpunkte in der HIV-Therapie sind somit dringend erforderlich.

HIV-Proteine werden analog zu Proteinen anderer Retroviren zuerst als lange Vorläufer Polyproteine gag, pol und env translatiert (C. Dickson et al. in RNA Tumor Viruses (Herausgeber: R. Weiss, N. Teich, H. Varmus und J. Coffin) 2nd Ed., revised, Seite 513 - 648, Cold Spring Harbor Laboratory, Cold Spring Harbor, NY) und erst anschließend proteolytisch zu den Strukturproteinen (p17 (MA), p24 (CA), p7 (NC) und p6), den Enzymen (Protease (PR), Reverse Transkriptase (RT) und Integrase (IN)), und den Hüllproteinen (gp120 (SU) und gp41 (TM)) prozessiert (Nomenklatur: J. Leis et al., J. Virol, 62, (1988), (1808-1809). Man nimmt an, daß die Spaltung der gag und pol Polyproteine durch eine viral codierte Protease bewirkt wird. Mutationen innerhalb der die Protease codierenden Region führen zu nicht infektiösen Viruspartikeln (N.E. Kohl et al. Proc. Natl. Acad. Sci. USA 85, (1988), (4686-4690).

Die HIV-Protease besteht aus 99 Aminosäuren und spaltet sich offensichtlich selbst durch Hydrolyse der beiden Phe-Pro-Bindungen in den Positionen 68 - 69 bzw. 167 - 168 aus dem pol Polyprotein heraus (M. C. Graves, J. J. Lim, E. P. Heimer und R. A. Kramer Proc. Natl. Acad. Sci. USA 85 (1988); 2449 - 2453; J. Hansen, S. Billich, T. Schulze, S. Sukrow und K. Mölling, EMBO J. 7 (1988), 1785 - 1791; E. P. Lillehoj et al., J. Virology 62 (1988) 3053 - 3058; J. Schneider und S. B. H. Kent, Cell 54 (1988) 363 - 368).

In der Literatur sind bereits einige Inhibitoren der HIV-Protease bekannt. Erster Vertreter war das Pepstatin A mit einem IC₅₀-Wert von ca. 0,5 mmol/l (1. Katoh, T. Yasunaga, Y. Ikawa und Y. Yoshinaka, Nature, 329, (1987), 654 - 656). Inzwischen sind einige weitere Inhibitoren beschrieben (siehe z. B. A. G. Tomaselli et al., Chim. Oggi 9(5), (1991), 6 - 27, EP 0428849, EP 0435059).

Es wurde nun eine neue Strukturklasse gefunden, die im Enzymtest hochwirksam die HIV-Protease hemmt.

Die vorliegende Erfindung betrifft Verbindungen der Formel 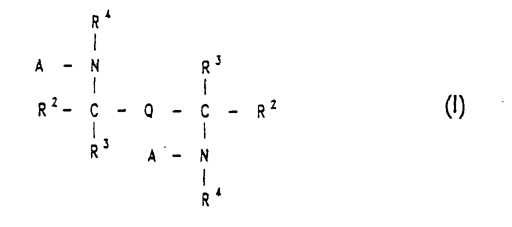 worin
- Q: für einen Rest der Formel IIa steht
- D: für R¹ oder einen Rest der Formeln V oder VI steht; 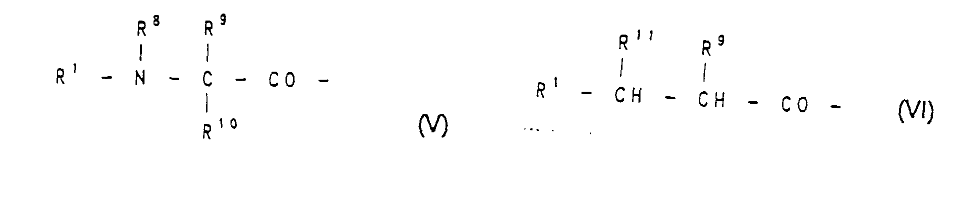
- R¹: Wasserstoff, (C₁-C₆)-Alkylsulfonyl, Phenyl-(C₁-C₂)-alkyl, Triphenylmethyl, (C₁-C₆)-Alkoxycarbonyl oder Phenyl-(C₁-C₂)-alkoxycarbonyl,
- R²: Wasserstoff, Phenyl, Benzyl, Methyl, Ethyl, Isopropyl, n-Propyl, n-Butyl, Isobutyl, sec.Butyl, Pentyl- oder Cyclohexylmethyl;
- R³, R⁴, R⁸, R¹⁰ und R¹¹: Wasserstoff,
- R⁵: Wasserstoff oder (C₁-C₄)-Alkyl,
- R⁶: Sauerstoff und
- R⁹: Wasserstoff, Isopropyl, sec.-Butyl, Benzyl, Carboxymethyl, 1-Naphthylmethyl, 2-(Methylthio)ethyl oder Indol-2-yl-methyl bedeüten
sowie deren physiologisch verträgliche Salze.

Die Chiralitätszentren in den Verbindungen der Formel (1) können die R-, S- oder R,S-Konfiguration aufweisen.

Alkyl kann geradkettig oder verzweigt sein. Entsprechendes gilt für davon abgeleitete Reste, wie z. B. Alkoxy, Alkylthio, Alkylamino, Dialkylamino, Alkanoyl und Aralkyl.

Unter Cycloalkyl werden auch alkylsubstituierte Reste, wie z. B. 4-Methylcyclohexyl oder 2,3-Dimethylcyclopentyl verstanden.

Unter Salzen von Verbindungen der Formel (1) sind insbesondere pharmazeutisch verwendbare oder nicht-toxische Salze zu verstehen.

Solche Salze werden beispielsweise von Verbindungen der Formel (I), welche saure Gruppen, z. B. zusätzlich Carboxy, enthalten, mit Alkali- oder Erdalkalimetallen gebildet, wie z. B. Na, K, Mg und Ca, sowie mit physiologisch verträglichen organischen Aminen, wie z. B. Triethylamin und Tris- (2-hydroxy-ethyl)- amin.

Verbindungen der Formel I, welche basische Gruppen, z. B. eine Aminogruppe oder eine Guanidinogruppe enthalten, bilden mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure und p-Toluolsulfonsäure, Salze.

Phosphinat-Pro-Drugs sind z. B. beschrieben in H. Bundgaard, "Design of Prodrugs", Elsevier, Amsterdam 1985, S. 70ff. Beispiele für solche Pro-Drug-Formen sind Glycerylester, 1,2-Difettsäureglyceryltriester, O-Acyloxyalkylester und 1 -Methyl-2-nitroethylester.

Pharmazeutisch verträgliche Kationen sind vorzugsweise Natrium, Kalium, Magnesium, Aluminium, Lithium, Ammonium und Triethylammonium.

Die vorliegende Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel 1 mit einer endständigen Carboxylgruppe besitzen z. B. die nachstehenden Formeln:

D - OH (VIII)

D - E - OH (IX)

Fragmente einer Verbindung der Formel I mit einer endständigen Aminogruppe besitzen z. B. die nachstehenden Formeln:

H - Z - H (XV)

H- E-Z-E -H (XVIb)

wobei Z für einen Rest der Formel (XIX) steht:

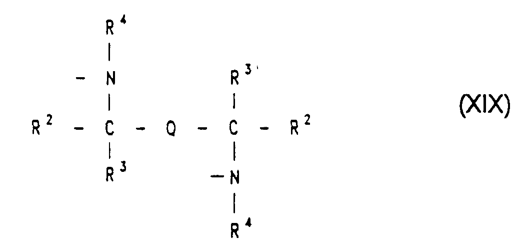

Im Falle, daß die beiden an Q gebundenen Rest verschieden sind, können auch andere Fragmente außer denen der Formeln XV bis XVIII, die eventuell an einer endständigen Aminogruppe geschützt sind, zum Einsatz kommen.

Methoden, die zur Herstellung einer Amidbindung geeignet sind, werden z. B. in Houben-Weyl, Methoden der organischen Chemie, Band 15/2; Bodanszky et al., Peptide Synthesis. 2nd ed. (Wiley & Sons, New York 1976) oder Gross, Meienhofer,
The Peptides: Analysis, synthesis, biology (Academic Press, New York 1979) beschrieben. Vorzugsweise werden die folgenden Methoden herangezogen: Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid (DCC) oder mit n-Propanphosphonsäureanhydrid (PPA) und die Gemischt-Anhydrid-Methode mit Pivaloylchlorid oder Chlorameisensäureethylester oder -isobutylester, oder Kupplung mit Phosphonium-Reagenzien, wie Benzotriazol-1-yl-oxy-tris-(dimethylamino-phosphonium-hexafluorophosphat (BOP) oder Uronium-Reagenzien, wie 2-(1H-Benzotriazol-1-yl)-1,1,3,3-tetramethyluronium tetrafluorobarat (TBTU).

Fragmente der Formel (VIII) sofern sie unter
b) Formel (VI) bzw (VI*) fallen, werden z. B. ausgehend von den entsprechenden Aminosäuren synthetisiert, wobei deren Chiralitätszentrum erhalten bleibt. Diazotierung bei - 20 °C bis 50 °C in verd. Mineralsäuren führt zu α-Bromcarbonsäuren oder über die Milchsäuren zu α-Trifluormethansulfonyloxy-Carbonsäuren die mit einem R¹ und R¹¹ bzw. R^{1*} und R^{11*} tragenden Nucleophil umgesetzt werden können, oder werden z. B. ausgehend von Malonestern hergestellt, deren Alkylierung mono- oder disubstituierte Malonester liefert, die nach Verseifung durch Decarboxylierung in die gewünschten Derivate überführt werden.

Fragmente der Formeln (IX), (X), (XI), (XII) und (XIII), (XIV) und (XIVa) werden nach den allgemeinen, bekannten Methoden zur Herstellung von Aminosäuren und Peptiden synthetisiert.

Fragmente der Formel XV werden nach bekannten Verfahren synthetisiert (K. Sasse in Houben-Weyl, Methoden der organischen Chemie, Band 12/1, Georg Thieme Verlag, Stuttgart, 1963; U.-H. Felcht in Houben-Weyl, Methoden der organischen Chemie, Band 12/E/2, Georg Thieme Verlag, Stuttgart, 1982; D. Redmore in Griffiths, Ed., Phosphorous Chemistry, Vol. 8, S. 515). Vorzugsweise werden folgende Methoden herangezogen:

### 1) Synthese der Verbindungen der Formel XVa

B Z B (XVa)

wobei B für Schutzgruppen, insbesondere Benzyloxycarbonyl steht, durch Umsetzung von substituierten a-Amino-phosphonig-säureestern, welche nach bekannten Methoden hergestellt werden (J. Org. Chem. 53 (1988) 4500) z. B. 1-Benzyloxy-carbonylamino-2-phenyl-ethyl-phosphonigsäureethylester mit nach bekannten Methoden hergestellten substituierten a-Aminoaldehyden, z. B. N-Benzyloxycarbonylamino-L-phenylalaninal (Lit. Tetrahedron Lett 22 (1988) 3815; ibid 31 (1990) 7359).

Die Umsetzungen erfolgen in einem organischen Lösungsmittel, bevorzugt Chloroform durch Basenkatalyse, bevorzugt mit Triethylamin bei - 78 °C bis 100 °C bevorzugt bei ca. 60 °C.

Peptidanaloge dieser Art können nach bekannten Verfahren hergestellt werden, die beispielsweise folgenden Literaturstellen entnommen werden können:
A. F. Spatola in "Chemistry and Biochemistry of Amino Acids Peptides and Proteins" 1983 (B. Weinstein et al. eds.) Marcel Dekker, New York, S. 267 (Reviewartikel); J. S. Morley, Trends Pharm Sci. (1980) S. 463 - 468 (Reviewartikel);
D. Hudson et al., Int. J. Pept. Prot. Res. (1979), 14, 177-185 (-CH₂NH-, -CH₂CH₂-);
A. F. Spatola et al., Life Sci. (1986), 38, 1243- 1249 (-CH2-S-);
M. M. Hann, J. Chem. Soc. Perkin Trans.l (1982) 307 - 314 (-CH=CH-, cis und trans);
J. K. Whitesell et al., Chirality 1, (1989) 89 - 91 (-CH=CH-trans)
R. G. Almquist et al., J. Med. Chem. (1980), 23, 1392- 1398 (-COCH₂-);
C. Jennings-White et al., Tetrahedron Lett. (1982) 23, 2533 (-COCH₂-);
M. Szelke et al., EP-A 45665 (1982), CA: 97: 39405 (-CH(OH)CH₂-);
M. W. Holladay et al., Tetrahedron Lett. (1983) 24, 4401 - 4404 (-CH(OH)CH₂-);
V. J. Hruby, Life Sci. (1982), 31, 189 - 199 (-CH₂-S-);
N. E. Jacobsen, P.A. Barlett, J. Am. Chem. Soc. (1981) 103, 654 - 657 (-P(O)(OR)NH-).

Die zur Herstellung von Verbindungen der Formel erforderlichen Vor- und Nachoperationen wie Einführung und Abspaltung von Schutzgruppen sind literaturbekannt und sind z. B. in T. W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt, indem man z. B. eine Verbindung der Formel 1 mit einer basischen Gruppe mit einer stöchiometrischen Menge einer geeigneten Säure oder Verbindungen der Formel I mit einer sauren Gruppe mit einer stöchiometrischen Menge einer geeigneten Base umsetzt. Stereoisomerengemische, insbesondere Diastereomerengemische, die gegebenenfalls bei der Synthese von Verbindungen der Formel 1 anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf. Insbesondere hemmen sie HIV-Protease. Ihre enzyminhibitorische Wirkung, die im milli- bis subnanomolaren Bereich liegt, kann wie folgt bestimmt werden.

### Testprinzip:

Als Substrat der HIV-Protease diente bisher unter anderen das Heptapeptid: H-Ser-Phe-Asn-Phe-Pro-Gln-Ile-OH (P. L. Darke et al., Biophys. Res. Commun. 156 (1988) 297 - 303). Die HIV-Protease spaltet das Substrat dabei zwischen dem zweiten Phe und Pro.

Überraschenderweise wurde nun gefunden, daß Substitution von Prolin durch 5-Oxaprolin in dieser Sequenz zu einem Substrat führt, das wesentlich schneller von der HIV-Protease gespaltet werden kann und damit eine schnellere Analyse mit geringerem Enzymbedarf erlaubt.

Allgemeine Vorschrift für die Austestung von Inhibitoren der HIV-Proteasen:
a) Herstellung der Substratlösung:
   2 mg H-Ser-Phe-Asn-Phe-Opr-Gln-lle-OH (H-Opr-OH = 5-Oxaprolin) werden in 1 ml MGTE 15-Puffer gelöst (evtl. Anwendung von Ultraschall) und anschließend über einen Sterilfilter (0,45 *µ*m) filtriert.
b) Herstellung der Inhibitorlösung:
   Vom Inhibitor werden das 2,5-fache der gewünschten Molarität je ml Lösung eingewogen und mit DMSO (10 % des Endvolumens) gelöst. Man verdünnt mit MGTE 15-Puffer bis zum Endvolumen und filtriert über Sterilfilter (0,45 *µ* m).
c) Herstellung der Proteaselösung:
   5 *µ*l der HIV-Proteaselösung werden nach Bedarf mit MGTE25-Puffer verdünnt.
d) Testdurchführung:
   Man pipettiert je 10 *µ*l der Substratlösung in Reagenzgläser (16 x 100) mit Schraubdeckel. Zum Blindversuch werden 10 *µ*l MGTE 15-Puffer, der 10 % DM50 enthält, pipettiert. Die übrigen Reagenzgläser werden mit je 10 *µ*l der Inhibitorlösungen versetzt. Man inkubiert 5 - 10 Minuten bei 37 °C und gibt dann zu jeder Probe 5 *µ*l der Proteaselösung. Nach 2 Stunden Reaktion bei 37 °C werden von jeder Probe 10 oder 20 *µ* l (je nach Empfindlichkeit des HPLC-Gerätes) abpipettiert, in Mikrovials gefüllt und mit 120 *µ*l des HPLC-Laufmittels verdünnt.
e) Bedingungen für die HPLC-Analyse:
   - Laufmittelsystem:: 80 % 0,1 M Phosphorsäure pH 2,5 20 % (w/w) Acetonitri I

   Säule: Merck ® LICHROSORB RP18 (5 *µ*m) 250 x 4
   Fluß: 1 m1/min
   Temperatur der Säule: 42°C
   Detektorparameter: 215 nm, 0,08 AUF, 18,2°C
   Analysenzeit: 11 Minuten
   Retentionszeit des Substrates: 8,1 Minuten
   Retentionszeit des N-terminalen Tetrapeptids: 3,9 Minuten
f) Benötigte Lösungsmittel:
   1) MGTE 15-Puffer:
      20 mM Morpholinoethansulfonsäure (MES)
      15 % (w/v) Glycerin
      0,1 % (v/v) Triton X 100
      5 mM EDTA
      0,5 M NaCl
      1 mM Phenylmethylsulfonylfluorid (PMSF)
   2) MGTE 25-Puffer:
      Zusammensetzung ähnlich wie beim MGTE 15-Puffer mit folgender Abweichung:
      25 % (w/v) Glycerin,
      zusätzlich 1 mM Dithiothreit (DTT)

      In einen Erlenmeyerkolben wiegt man MES, EDTA, NaCI, DTT und PMSF ein, löst in wenig Wasser und stellt auf pH 6 ein. In einen Meßkolben wiegt man die entsprechende Menge Glycerin ein und pipettiert ® Triton X 100 dazu. Man überführt die wäßrige Lösung in den Meßkolben und füllt mit Wasser auf.
   3) HPLC-Laufmittel:
      Man stellt sich aus ortho-Phosphorsäure (FLUKA puriss. p.a.) eine 0,1 M Lösung her. Mit Triethylamin (FLUKA puriss. p.a.) wird diese Lösung genau auf pH 2,5 eingestellt. Das Gewicht der Lösung wird bestimmt und die entsprechende Menge Acetonitril (Abzug!) zugewogen. Gut durchmischen und ca. 5 Minuten mit Helium 5,0 entgasen.
g) Auswertung:

Unter den hier gewählten Bedingungen trennen sich die Heptapeptide von dem bei der enzymatischen Spaltung entstehenden N-terminalen Tetrapeptid. Der %-Gehalt des Tetrapeptid-peaks in Bezug auf Summe Tetrapeptid + Heptapeptid entspricht der Spaltrate. Die nachfolgenden IC₅₀-Werte geben an, bei welcher Inhibitorkonzentration die Spaltrate halbiert ist:

| Beispiel | IC₅₀ |
|---|---|
| Nr. 3 | 70 nM |
| 4 | 1,3 nM |
| 7 | 15 nM |
| 8 | 0,5 nM |
| 10 | 4,2 nM |

Das Zielpeptid wurde mit einem Peptid-Synthesizer Modell 430 A der Fa. Applied Biosystems unter Verwendung der Fmoc-Methode an einem mit Fmoc-Ile-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

Man verwendet folgende Aminosäure-Derivate: Fmoc-Gln-OH, Fmoc-Opr-OH, Fmoc-Phe-OObt, Fmoc-Asn-OH und Fmoc-Ser(tBu)-OObt. Zur Synthese von FmocOpr-OH wurde H-Opr-OtBu nach der Methode von Vasella et al. (J.C.S. Chem. Comm. 1981, 97 - 98) synthetisiert und mit Fmoc-OSu in 15 Dioxan/Wasser (1 : 1) in Gegenwart von NaHCO₃ umgesetzt. Die anschließende Spaltung des tert.-Butylesters mit Trifluoressigsäure liefert Fmoc-Opr-OH.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 molaren Lösung von Diisopropylcarbodiimid in DMF. Die HOObt-Ester der anderen Aminosäuren wurden in 6 ml NMP gelöst und dann ebenso wie die in situvoraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach° mit Essigester digeriert und zentrifugiert.

Der verbliebene Rückstand wurde an einem alkylierten Dextrangel mit 10 %iger Essigsäure chromatographiert. Die das reine Peptid enthaltende Fraktion wurde vereinigt und gefriergetrocknet.
- Massenspektrum (FAB):: 854 (M+H+)
- Aminosäureanalyse Asp:: 0,98; 5er: 0,80; Glu: 1,00; Ile: 1,05; Phe: 2,10; NH₃: 1,76.

Die Erfindung betrifft auch die Verwendung der Verbindungen der Formel 1 als Heilmittel und pharmazeutische Präparate, die diese Verbindung enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z. B. in Frage: Wasser, physiologische Kochsalzlösungen oder Alkohole, z. B. Ethanol, Propandiol oder Glycerin, daneben auch Zuckerlösungen wie Glucose- oder Mannitlösungen, oder auch eine Mischung aus den verschiedenen genannten Lösungsmitteln.

Ebenfalls möglich ist der Einsatz von injizierbaren Retardzubereitungen. Als Arzneiformen können z. B. ölige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin sein können.

### Verzeichnis der Abkürzungen:

- Boc: tert.-Butyloxycarbonyl
- Chg: Cyclohexylglycyl
- d: Dublett
- DC: Dünnschichtchromatographie
- DCC: Dicyclohexylcarbodiimid
- DCM: Dichlormethan
- DMF: Dimethylformamid
- DMAP: 4-Dimethylaminopyridin
- DMSO: Dimethylsulfoxid
- EDAC: I-(3-Dimethylamino-propyl)-3-ethyl-carbodiimid Hydrochlorid
- EE: Essigsäureethylester
- FAB: Fast atom bombardment
- HOBt: Hydroxybenzotriazol
- i. Vac.: im Vakuum
- m: Multiplett
- M: Molekularpeak
- NEM: N-Ethylmorpholin
- Npg: Neopentylglycyl
- MS: Massenspetrum
- PPA: n-Propylphosphonsäureanhydrid
- RT: Raumtemperatur
- s: Singulett
- Schmp.: Schmelzpunkt
- t: Triplett
- Tbg: tert.-Butylglycyl
- TBTU: 2-(1H-Benzotriazol-1-yl)-1,1,3,3-tetramethyluronium tetrafluoroborat
- THF: Tetrahydrofuran
- Thia: 2-Thienylalanyl
- Z: Benzyloxycarbonyl

Die sonstigen für Aminosäuren verwendeten Abkürzungen entsprechen dem in der Peptidchemie üblichen Drei-Buchstaben-Code (wie er z. B. in Eur. J. Biochem. 138, (1984), 9 - 37 beschrieben ist). Falls nicht ausdrücklich anders angegeben, handelt es sich immer um eine Aminosäure der L-Konfiguration.

Die nachstehenden Beispiele dienen zur Erläuterung der vorliegenden Erfindung, ohne daß diese darauf beschränkt wäre.

### Beispiel 1

### a.) 1-Diphenylmethylamino-2-phenylethyl-phosphonigsäure

Zu einer unter Argon auf 100 °C erhitzten Suspension von 31,85 g (0,124 mol) Diphenylmethyl-aminohypophosphit in 350 ml Dioxan, hergestellt durch Mischen equimolarer Mengen von Diphenylmehtylamin und Hypophosphoriger Säure (100 %) in Ethanol, wird langsam eine Lösung von 17,59 g (0,124 mol) Phenylacetaldehyd in 20 ml Dioxan getropft. Die Reaktionslösung wird 1 h bei dieser Temperatur gerührt. Das gebildete Reaktionswasser wird durch azeotrope Destillation mit 280 ml Dioxan entfernt. Nach Abkühlen der Reaktionslösung und Verdünnen mit 150 ml Ethanol wird das langsam ausfallende Produkt abgesaugt, mit Ethanol/Diethylether gewaschen und über P2O5 getrocknet.
Ausbeute: 9,84 g (22,5 %); Schmp.: 209 - 211 °C; MS: 352 (M+H)+, 286; 167

### b.) 1-Amino-2-phenylethylphosphonigsäure

9,9 g (0,028 mol) 1-Diphenylmethylamino-2-phenylethylphosphonigsäure werden in 50 ml 40 %iger Bromwasserstoffsäure 2 h auf 100 - 105 °C erhitzt. Anschl. wird die Reaktionslösung i. V. bis zur Tockne eingedampft und der Rückstand in 50 ml Wasser aufgenommen. Die wässrige Lösung wird mehrmals mit Diethylether gewaschen und zur Trockne eingedampft. Der Rückstand wird in 60 ml Ethanol gelöst und die Lösung wird bis zum Ausfallen des Produktes mit Propylenoxid versetzt. Nach Stehen über Nacht wird der Niederschlag abgesaugt, mit Ethanol/Diethylether gewaschen und getrocknet.
Ausbeute: 4,23 g (82 %); Schmp.: 225 - 226 °C; MS: 186 (M+H)+; 120

### c.) 1-Benzyloxycarbonylamino-2-phenylethylphosphonigsäure

Zu einer Suspension von 1,76 g (9,5 mmol) 1-Amoni-2-phenylethylphosphonigsäure in 15 ml 1 M NaOH, 5 ml Wasser und 5 ml Dioxan werden innerhalb von 30 min. bei 0 °C 2,25 ml (14,25 mmol) Chlorameisensäurebenzylester getropft. Während weiteren 2,5 h Rühren bei 0 °C wird der pH-Wert der Mischung mit 1 M NaOH (ca. 10 ml) bei 9 - 10 gehalten. Nach Entfernen der Kühlung wird über Nacht bei Rt gerührt. Anschl. wird die Reaktionslsg. mehrmals mit Diethylether gewaschen. Die wässrige Phase wird bei 0 - 5 °C mit 6 M HCI auf pH 2 gebracht und mehrmals mit Ethylacetat extrahiert. Die vereinigten Extrakte werden mit ges. NaCl-Lösung gewaschen, mit Na₂SO₄ getrocknet, filtriert und i. V. einrotiert.
Es verbleibt ein fester Rückstand, der aus Ether/Petrolether umkristallisiert wird.
Ausbeute: 2,62 g (86 %); MS 342 (M+Na)+, 320 (M+H)+; 254
Schmp.: 136 - 137 °C

### d.) 1-Benzyloxycarbonylamino-2-phenylethylphosphonigsäureethylester

Zu einer Suspension von 7,98 g (25 mmol) 1-Benzyloxycarbonylamino-2-phenylethylphosphonigsäure und 2,53 ml (25 mmol) Chlorameisensäureethylester unter Argon in 200 ml Chloroform werden bei Rt innerhalb von 20 min. 2,24 ml (25 mmol) Pyridin getropft. Die Reaktionslsg. wird für weitere 30 min. bei dieser Temperatur gerührt. Anschl. wird für 1 h auf 70 °C erwärmt. Nach Abdampfen des Lösungsmittels i. V. wird der Rückstand in Ethylacetat aufgenommen, mit Wasser und ges. NaCI-Lösung gewaschen, über Na₂SO₄ getrocknet, filtriert und i. V. eingedampft. Der verbleibende feste Rückstand wird aus Ether/Petrolether umkristallisiert.
Ausbeute: 7,6 g (88 %); MS: 348 (M+H)+; 254; 210

### e.) 2-Benzyloxycarbonyl-L-phenylalanin-N-methoxy-N-methylamid

Zu einer Lösung von 17,94 g (0,06 mol) Benzyloxycarbonylphenylalanin, 5,97 g (0,06 mol) N,O-Dimethyl-hydroxyamin-hydrochlorid, 8,79 g (0,06 mol) Hydroxyiminocyanessigsäureethylester und 19,68 g (0,06 mol) TOTU in 120 ml DMF werden bei 0 °C 31,8 ml (0,18 mol) N-Ethyl-diisopropylamin getropft. Die Mischung wird 1 h bei 0 °C und 3 h bei Rt gerührt. Das Lösungsmittel wird i. V. abrotiert, der Rückstand in Ethylacetat aufgenommen und die so erhaltene Lösung mehrmals mit 10 %iger Citronensäurelösung, 10 %iger KHCO₃-Lsg. und ges. NaCI-Lsg. gewaschen. Nach Trocknen über Na₂SO₄, Filtration und Einengen des Filtrates verbleiben 13,95 g (68 %) eines Öls.
MS: 343 (M+H)+

### f.) N-Benzyloxycarbonyl-L-phenylalaninal

4,35 g (12,71 mmol) 2-Benzyloxycarbonyl-L-phenylalanin-N-methoxy-N-methylamid gelöst in 40 ml Diethylether werden innerhalb von 30 min. zu einer bei 0 °C unter Argon gerührten Suspension von 0,965 g (25,42 mmol) Lithiumaluminiumhydrid in 140 ml Diethylether getropf. Nach weiteren 30 min. wird die Kühlung entfernt und 30 min. bei Rt gerührt. Die Mischung wird vorsichtig mit 50 ml Eiswasser versetzt und filtriert. Das Filtrat wird mit 10 %iger H₂SO₄ auf pH 4 gestellt und mehrmals mit Ether extrahiert. Die vereinigten Etherextrakte werden mit Wasser und ges. NaCl-Lösung gewaschen, über Na₂SO₄ getrocknet, filtriert und i. V. vom Lösungsmittel befreit.
Zurück bleibt das ölige Produkt.
Ausbeute: 3,41 g (95 %); MS: 284 (M+H)+; 254; 240; 210

### g.) 1-(2-Benzyloxycarbonylamino-1-hydroxy-3-phenylpropyl),1-(1-benzyloxycarbonylamino-2-phenylethyl)phosphinsäureethylester

Eine Lösung von 4,18 g (12 mmol) 1-Benzyloxycarbonylamino-2-phenylethylphosphonig-säureethylester, 3,41 g (12 mmol) N-Benyzloxycarbonyl-L-phenylalaninal und 0,85 ml (6 mmol) Triethylamin in 100 ml Chloroform unter Argon wird 20 h bei Rt gerührt. Anschl. werden nochmals 0,85 ml (6 mmol) Triethylamin zugesetzt. Die Mischung wird 9 h am Rückfluß gekocht. Nach Abrotieren des Lösungsmittels wird der Rückstand in 200 ml Ethylacetat gelöst und mehrmals mit Wasser und ges. NaCl-Lösung gewaschen. Die org. Phase wird mit Na₂SO₄ getrocknet, filtriert und i. V. einrotiert. Der Rückstand wird durch Chromatographie an Kieselgel (Ethylacetat/n-Heptan: 5/1) gereinigt.
Ausbeute: Isomer A 1,74 g (23 %); Isomer B 0,81 g (11 %)
MS: 631 (M+H)+; 240; 210

### Beispiel 2

### 1-(2-Amino-1-hydroxy-3-phenylpropyl),1-(1-amino-2-phenylethyl)phosphinsäureethylester dihydrochlorid

1,5 g (2,38 mmol) 1-(2-Benzyloxycarbonylamino-1-hydroxy-3-phenylpropyl),1-(1-benzyloxycarbonylamino-2-phenylethyl)phosphinsäureethylester (Isomer A) werden in 80 ml Methanol gelöst und mit 0,3 g Pd/C (10 %) während 3 h mit H2 hydriert, dabei wirx der pH-Wert der Reaktionslsg. mit methanol. HCI auf 3 gehalten. Der Katalysator wird abfiltiert und das Filtrat zur Trockne eingedampft. Das zurückbleibende Produkt wird aus Isopropanol/Ether umkristallisiert.
Ausbeute: 0,9 g (87 %); MS: 363 (M+H)+; 244; 120

### Beispiel 3

### 1-{2-[(Benzyloxycarbonyl-L-valyl)amino]-1-hydroxy-3-phenylpropyl},1-{1-[(benzyloxycarbonyl-L-valyl)amino]}-2-phenylethyl}phosphinsäureethylester

Zu einer Lösung von 440 mg (1 mmol) Verbindung aus Beispiel 2, 630 mg (2,5 mmol) Benzyloxycarbonylvalin, 360 mg (2,5 mmol) Hydroxyiminocyanessigsäureethylester und 820 mg (2,5 mmol) TOTU in 40 ml DMF werden bei 0 °C und 4 h bei Rt gerührt. Das Lösungsmittel wird i. V. abrotiert und der Rückstand in 100 ml Ethylacetat gelöst. Die Ethylacetatlösung wird mehrmals mit 10 %iger Citronensäurelsg., 10 %iger KHCO₃-Lsg. und ges. NaCl-Lsg. gewaschen, über Na₂SO₄ getrocknet, filtriert und i. V. zur Trockne eingedampft. Der feste Rückstand wird aus Ethylacetat umkristallisiert.
Ausbeute: 0,58 g (70 %); MS: 851 (M+Na)+, 829 (M+H)+

### Beispiel 4

### 1-{2-[(Benzyloxycarbonyl-L-valyl)amino]-1-hydroxy-3-phenylpropyl},1-{-[(benzyloxycarbonyl-L-valyl)amino]-2-phenylethyl}phosphinsäure

Zu einer Lösung von 124 mg (0,149 mmol) Verbindung aus Beispiel 3 in 5 ml Tetrahydrofuran unter Argon werden bei 0 °C 0,06 ml (0,447 mmol) Trimethylsilylbromid getropft. Nach 1 h bei dieser Temperatur und 24 h bei Rt wird die Lösung mit 2 ml Eiswasser versetzt und mit 50 ml Ethylacetat extrahiert, filtriert und i. V. eingedampft. Zurück bleibt ein zähes öliges Produkt. Die Reinigung erfolgt durch Chromatographie an Kieselgel (Ethylacetat/n-Heptan: 5/1)
Ausbeute: 25,6 mg (21,5 %); MS: 823 (M+Na)+; 801 (M+H)+

### Beispiel 5

### 1-[2-(L-Valyl-amino)-1-hydroxy-3-phenylpropyl],1-[L-valyl-amino)-2-phenylethyl]phosphinsäureethylester-dihydrochlorid

Synthese analog zu Beispiel 2 aus 1-{2-[(Benzyloxycarbonyl-L-valyl)amino]-1-hydroxy-3-phenylpropyl},1-{1-[(benzyloxycarbonyl-L-valyl)amino]-2-phenylethyl}phosphinsäureethylester.
Ausbeute: 0,24 g (90 %); MS: 561 (M+H)+; 462; 249

### Beispiel 6

### 1-{2-[(N-tert.butyloxycarbonyl-L-naphthylalanyl-L-valyl)amino]-1-hydroxy-3-phenylpropyl},1-{1-[(N-tert.butyloxycarbonyl-L-naphthylalanyl-L-valyl)amino]-2-phenyltheyl}phosphinsäureethylester

Eine Lösung von 470 mg (1,5 mmol) N-ter.-butyloxycarbonyl-L-naphthylalanin und 202 mg (1,5 mmol) 1-Hydroxybenzotriazol in 15 ml Tetrahydrofuran wird bei 0 °C mit 340 mg (1,64 mmol) 1,1'-Carbonyl-diimidazol versetzt. Die Mischung wird 15 min. bei 0 °C und 2 h bei Rt gerührt. Die so erhaltene Aktivesterlösung wird ebenfalls bei 0 °C zu einer Mischung aus 320 mg (0,5 mmol) Verbindung aus Beispiel 5 und 0,17 ml (1 mmol) N-Ethyl-diisopropylamin in 15 ml DMF gegeben, gefolgt von weiteren 0,34 ml (2 mmol) N-Ethyl-diisopropylamin. Anschl. wird das Reaktionsgemisch 2 h bei 0 °C und 4 h Rt gerührt. Das Lösungsmittel wird i. V. abrotiert und der Rückstand durch Chromatographie an Kieselgel (CH₂Cl₂/CH₃OH :
40/1) gereinigt.
Ausbeute: 360 mg (62 %); MS: 1178 (M+Na)+, 1156 (M+H)+

### Beispiel 7

### 1-{2-[(L-Naphthylalanyl-L-valyl)amino]-1-hydroxy-3-phenylpropyl},1-{1-[(L-naphthylalanyl-L-valyl)amino]-2-phenylethyl}phosphinsäureethylester bistrifluoracetat

Eine Lösung von 72 mg (0,06 mmol) aus Beispiel 6 erhaltene Verbindung in 2 ml Trifluoressigsäure wird 1 h bei Rt gerührt und anschl. i. V. zur Trocknung eingedampft. Das durch Zugabe von Ethylacetat ausgefällte Produkt wird abgesaugt mit Ether gewaschen und über P₂O₅ getrocknet.
Ausbeute: 56,7 mg (80 %); MS: 997,5 (M+Na)+, 955,5 (M+H)+

### Beispiel 8

### 1-{2-[(L-Naphthylalanyl-L-valyl)amino]-1-hydroxy-3-phenylpropyl},1-{1-[(L-naphthylalanyl-L-valyl)amino]-2-phenylethyl}phosphinsäure dihydrobromid

543 mg (0,47 mmol) erhaltene Verbindung aus Beispiel 7 werden mit 0,5 ml (3,81 mmol) Trimethylsilylbromid analog der in Beispiel 4 beschriebenen Methode behandelt. Das Rohprodukt wird durch Chromatographie an Kieselgel (CH₂Cl₂/CH₃OH : 5/1) gereinigt.
Ausbeute: 255 mg (50 %); MS: 965,8 (M+K)+; 927,4 (M+H)+

### Beispiel 9

### 1-{2-[((2S(tert.butyl-sulfonylmethyl)-3-(1-naphthyl)-propionyl)-L-valyl)amino]-1-hydroxy-3-phenylpropyi},1-{1-[((2S(tert.butyl-sulfonylmethyl)-3-(1-naphthyl)-propionyl)-L-valyl)amino]-2-phenylethyl}phosphinsäureethylester

Eine Lösung von 521 mg (1,56 mmol) (2S(tert.butyl-sulfonylmethyl)-3-(1-naphthyl)-propionsäure und 254 mg (1,56 mmol) 3-Hydroxy-4-oxo-3,4-dihydro-1,2,3-benzotriazin in 3 ml Tetrahydrofuran wird bei 0 °C mit 321 mg (1,56 mmol) N,N'-Dicyclohexylcarodiimid versetzt. Die Mischung wird 30 min. bei 0 °C und 20 min. bei Raumtemperatur gerührt. Nach Filtration, wird die erhaltene Aktivesterlösung bei 0 °C zu einer Mischung aus 330 mg (0,52 mmol) Verbindung aus Beispiel 5 und 0,18 ml (1,04 mmol) N-Ethyl-diisopropylamin in 30 ml DMF gegeben, gefolgt von weiteren 0,36 ml (2,08 mmol) N-Ethyl-diisopropylamin. Anschließend wird das Reaktionsgemisch 2 h bei 0 °C und 24 h bei Raumtemperatur gerührt. Das Lösungsmittel wird i. V. abrotiert und der Rückstand in 150 ml Ethylacetat aufgenommen. Die Ethylacetatlösung wird mehrmals mit 10 %iger Citronensäurelösung, 10 %iger KHCO₃-Lösung und ges. NaCl-Lösung gewaschen, über Natriumsulfat getrocknet, filtriert und i. V. zur Trocknung eingedampft. Die Reinigung erfolgt durch Chromatographie an Kieselgel
(Ethylacetat/n-Heptan : 5/1).
Ausbeute: Isomer A 0,21 g (34 %) Isomer B 0,15 g (24 %)
MS: 1215 (M+Na)+; 1193 (M+H)

### Beispiel 10

### 1-{2-[((2S (tert.butyl-sulfonylmethyl)-3-(1-naphthyl)-propionyl)-L-valyl)amino]-1-hydroxy-3-phenylpropyl},1-{1-[((2S(tert.butyl-sulfonylmethyl)-3-(1-naphthyl)-propionyl)-L-valyl)amino]-2-phenylethyl}phosphinsäure

180 mg Isomer A und 120 mg Isomer B aus Beispiel 9 werden mit Trimethylsilylbromid analog der in Beispiel 7 beschriebenen Methode behandelt. Die Rohprodukte werden durch Chromatographie an Kieselgel (CH₂Cl₂/CH₃OH : 5/1) gereinigt.
Ausbeute: 88 mg (50 %) Isomer A 55 mg (47 %) Isomer B
MS: 1209 (M+2Na-H)⁺; 1203 (M+K)⁺; 1187 (M+Na)⁺

## Patentansprüche

1. Verbindung der Formel I 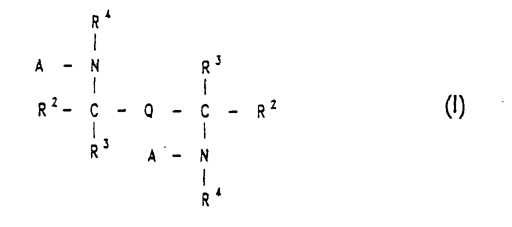 worin
Q für einen Rest der Formel IIa steht 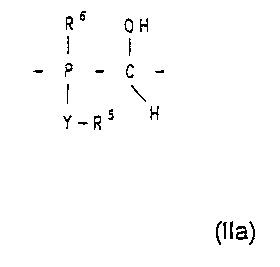
Y Sauerstoff ist,
A einen Rest der Formel IV bedeutet, wobei
D - (E)n - (IV)
E, Val, Phe, Ile oder Asp bedeuten und 0 oder 1 ist;
D für R¹ oder einen Rest der Formeln V oder VI steht; 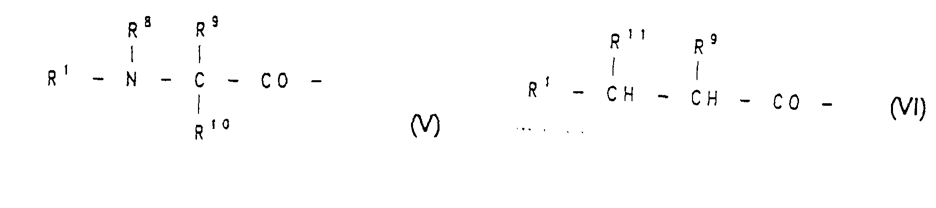
R¹ Wasserstoff, (C₁-C₆)-Alkylsulfonyl, Phenyl-(C₁-C₂)-alkyl, Triphenylmethyl, (C₁-C₆)-Alkoxycarbonyl oder Phenyl-(C₁-C₂)-alkoxycarbonyl,
R² Wasserstoff, Phenyl, Benzyl, Methyl, Ethyl, Isopropyl, n-Propyl, n-Butyl, Isobutyl, sec.Butyl, Pentyl- oder Cyclohexylmethyl;
R³, R⁴, R⁸, R¹⁰ und R¹¹ Wasserstoff,
R⁵ Wasserstoff oder (C₁-C₄)-Alkyl,
R⁶ Sauerstoff und
R⁹ Wasserstoff, Isopropyl, sec.-Butyl, Benzyl, Carboxymethyl, 1-Naphthylmethyl, 2-(Methylthio)ethyl oder Indol-2-yl-methyl
sowie deren physiologisch verträgliche Salze.

2. Verfahren zur Herstellung einer Verbindung der Formel 1 gemäß Anspruch 1 **dadurch gekennzeichnet, daß** man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

3. Verbindung der Formel I gemäß Anspruch 1 zur Anwendungab Heilmittel.

4. Verbindung der Formel I gemäß Ansprüch 1 zur Anwendungab Hemmer von retroviraler Proteasen.

5. Verwendung einer Verbindung der Formel I gemäß Anspruch 1 zür Herstellüng von pharmazeütischen Mitteln zür Behandlung des "erworbenen Immunschwäche-Syndroms".

6. Pharmazeutisches Mittel enthaltend eine Verbindung gemäß Anspruch 1.

## Claims

1. A compound of the formula I 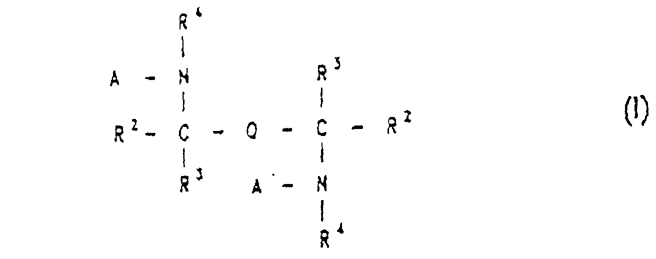 in which
Q is a radical of the formula IIa 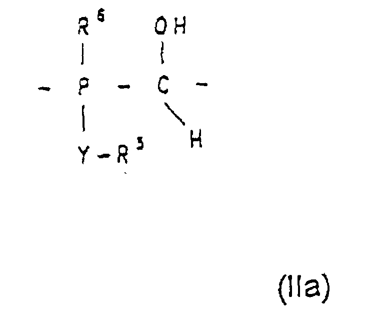
Y is oxygen,
A is a radical of the formula IV
D - (E)n - (IV)
where
E is Val, Phe, Ile or Asp, and
n is 0 or 1;
D is R¹ or a radical of the formulae V or VI
R¹ is hydrogen, (C₁-C₆)-alkylsulfonyl, phenyl-(C₁-C₂)-alkyl, triphenylmethyl, (C₁-C₆)-alkoxycarbonyl or phenyl-(C₁-C₂)-alkoxycarbonyl,
R² is hydrogen, phenyl, benzyl, methyl, ethyl, isopropyl, n-propyl, n-butyl, isobutyl, sec-butyl, pentyl or cyclohexylmethyl,
R³, R⁴, R⁸, R¹⁰ and R¹¹ are hydrogen,
R⁵ is hydrogen or (C₁-C₄)-alkyl,
R⁶ is oxygen, and
R⁹ is hydrogen, isopropyl, sec-butyl, benzyl, carboxymethyl, 1-naphthylmethyl, 2-(methylthio)ethyl or indol-2-ylmethyl,
and the physiologically tolerated salts thereof.

2. A process for preparing a compound of the formula I as claimed in claim 1, wherein a fragment having a terminal carboxyl group, or a reactive derivative of this fragment, is coupled to a corresponding fragment having a free amino group, (a) protective group(s) which has/have, where appropriate, been temporarily introduced to protect further functional groups is/are eliminated, and the compound thus obtained is, where appropriate, converted into its physiologically tolerated salt.

3. A compound of the formula I as claimed in claim 1 for use as a medicine.

4. A compound of the formula I as claimed in claim 1 for use as an inhibitor of retroviral proteases.

5. The use of a compound of the formula I as claimed in claim 1 for preparing pharmaceutical compositions for the treatment of "acquired immune deficiency syndrome".

6. A pharmaceutical composition containing a compound as claimed in claim 1.

## Revendications

1. Composé de formule 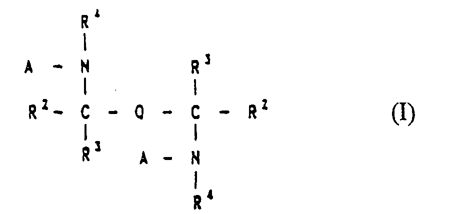 dans laquelle
Q représente un reste de formule IIa 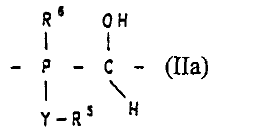
Y est un atome d'oxygène,
A représente un reste de formule (IV) D-(E)ₙ- (IV) dans laquelle
E est Val, Phe, Ile ou Asp, et
n est 0 ou 1 ;
D représente R¹ ou un reste de formule V ou VI 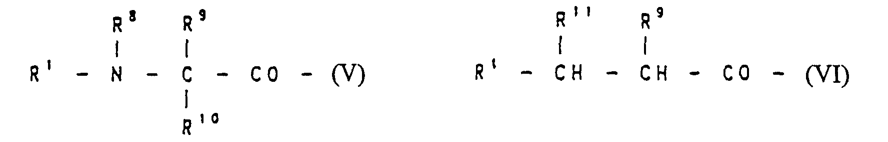
R¹ représente un atome d'hydrogène ou un reste alkylsulfonyle en C₁-C₆, phényl(alkyle en C₁-C₂), triphénylméthyle, (alcoxy en C₁-C₆)carbonyle ou phényl(alcoxy en C₁-C₂)carbonyle;
R² représente un atome d'hydrogène ou un groupe phényle, benzyle, méthyle, éthyle, isopropyle, n-propyle, n-butyle, isobutyle, sec-butyle, pentyle ou cyclohexylméthyle;
R³, R⁴, R⁸, R¹⁰ et R¹¹ sont des atomes d'hydrogène;
R⁵ représente un atome d'hydrogène ou un reste alkyle en C₁-C₄;
R⁶ est un atome d'oxygène et
R⁹ représente un atome d'hydrogène ou un groupe isopropyle, sec-butyle, benzyle, carboxyméthyle, 1-naphtylméthyle, 2-(méthylthio)éthyle ou indol-2-ylméthyle; et ses sels physiologiquement acceptables.

2. Procédé de préparation d'un composé de formule I selon la revendication 1, **caractérisé en ce que** l'on couple un fragment à groupe carboxyle terminal ou son dérivé réactif avec un fragment correspondant à groupe amino terminal, on sépare éventuellement un ou des groupes protecteurs introduits temporairement pour la protection d'autres groupes fonctionnels, et on transforme éventuellement le composé ainsi obtenu en son sel physiologiquement acceptable.

3. Composé de formule I selon la revendication 1 à utiliser comme médicament.

4. Composé de formule I selon la revendication à utiliser comme inhibiteur de protéases rétrovirales.

5. Utilisation d'un composé de formule I selon la revendication 1 pour la préparation de compositions pharmaceutiques pour le traitement du sida.

6. Composition pharmaceutique contenant un composé selon la revendication 1.
